# EUROPEAN PATENT APPLICATION

(11) **EP 1 279 434 A2**
(43) Date of publication of application: **29.01.2003**
(21) Application number: 02016613.8
(22) Date of filing: 25.07.2002
(51) Int. Cl.: B01J 19/00, C12Q 1/68

(54) **Hybridization substrate, method of manufacturing same, and method of use for same**

(30) Priority: 27.07.2001 JP 2001228374
(71) Applicant: Riken, Wako-shi, Saitama 351-0198 (JP)
(72) Inventor: Nakamura, Fumio, Otsu-shi, Shiga 520-0842 (JP); Hara, Masahiko, c/oRiken, Wako-shi, Saitama 351-0198 (JP); Nakajima, Ken, c/oRiken, Wako-shi, Saitama 351-0198 (JP); Ito, Eisuke, Wako-shi, Saitama 351-0198 (JP)
(74) Representative: Gervasi, Gemma, Dr.

(57) **Abstract**

Provided is a hybridization substrate in which DNA strands having a double-strand portion and a single-strand portion are immobilized on a substrate surface, wherein the double-strand portion side of said DNA strands is immobilized on said substrate surface. The invention relates to a method in which target DNA is contacted with the surface of the above substrates on which DNA strands have been immobilized to test for complementarity between the target DNA and the single-strand portion of said DNA strands having a double-strand portion and a single-strand portion.

## Description

### Technical Field

The present invention relates to a hybridization substrate, a method of manufacturing the same, and a complementarity test method employing the same.

### Background Technology

In genetic diagnosis, the identification of pathogenic bacteria, the detection of SNPs, and the like, a nucleotide probe is employed to detect a given nucleotide (target nucleotide). The nucleotide probe is mixed with the target nucleotide and the presence or absence of hybridization between the nucleotide probe and the target nucleotide is detected, for example, by means of a label such as fluorescent labeling on the nucleotide probe.

Since this nucleotide probe can be readily synthesized with a DNA synthesizer, DNA probes are primarily employed. From the perspective of ease of detection of the nucleotide probe hybridizing with the target nucleotide, fluorescent labeling is often employed, but in place of fluorescent labeling, RI is sometimes employed.

In recent years, DNA chips and DNA microarrays have been developed in which the nucleotide probes have been immobilized on substrates and are employed to detect target nucleotides.

In the manufacture of DNA chips and DNA microarrays, it is necessary to immobilize DNA on a substrate. DNA immobilization is accomplished by, for example, bonding a thiol to single-strand DNA to obtain thiolated DNA, which is then immobilized on a metal substrate. However, DNA immobilized by this method ends up assuming a collapsed structure on the substrate. Thus, there are problems in that both surface coverage and activity diminish substantially.

By contrast, numerous attempts to introduce an alkyl chain between the single-strand DNA and the thiol to enhance surface coverage and activity have been reported (For example, see J. Am. Chem. Soc. 1998, 120, 9787-9792).

However, this method is difficult to apply in practice due to the significant effort and cost that must be expended to modify the single-strand DNA (incorporate a long alkyl chain), requiring the incorporation of a mixture on the surface.

Accordingly, an object of the present invention is to provide a hybridization substrate in which a DNA strand is immobilized on the substrate surface enabling to enhance surface coverage and activity, a method of manufacturing the same, and a complementarity test method employing the same.

The invention solving the above-described problems is as follows.
A hybridization substrate in which DNA strands having a double-strand portion and a single-strand portion are immobilized on a substrate surface, wherein the double-strand portion side of said DNA strands is immobilized on said substrate surface.
The substrate according to claim 1 wherein said substrate is a metal substrate or a substrate having a metal coating, and said DNA strands have been immobilized on the metal surface of said substrate by means of a sulfur atom.
The substrate according to claim 1 wherein said substrate is a glass or silicon substrate and said DNA strands have been immobilized on the surface of said substrate by means of a sulfur atom.
The substrate according to any of claims 1 to 3 wherein the number of nucleic acids of said double-strand portion ranges from 10 to 80, and the number of nucleic acids of said single-strand portion ranges from 20 to 90.
The substrate according to any of claims 1 to 4 wherein in addition to said DNA strands having a double-strand portion and a single-strand portion, DNA strands having only a double-strand portion have been further immobilized on the surface of said substrate.
The substrate according to claim 5 wherein said substrate is a metal substrate or a substrate having a metal coating and said DNA strands having only a double-strand have been immobilized on said metal surface of said substrate by means of a sulfur atom.
The substrate according to claim 5 wherein said substrate is a glass substrate or silicon substrate and said DNA strands having only a double-strand portion have been immobilized on the surface of said substrate by a sulfur atom.
The substrate according to any of claims 5 to 7 wherein the immobilization ratio of said DNA strands having a double-strand portion and a single-strand portion to DNA strands having only a double-strand portion ranges from 99:1 to 1:99.
The substrate according to claim 2 or claim 6 wherein said metal substrate or metal coating is made of gold.
A method of manufacturing the substrate of claim 2 in which DNA strands having a double-strand portion and a single-strand portion, with a thiol group being present on the terminal of said double-strand portion, are contacted with the metal surface of a metal substrate or substrate having a metal coating to immobilize said DNA strands on said metal surface.
A method of manufacturing the substrate of claim 6 in which DNA strands having a double-strand portion and a single-strand portion, with a thiol group being present on the terminal of said double-strand portion, and DNA strands comprised of only a double-strand portion on the terminal of which a thiol group is present are contacted with the metal surface of a metal substrate or substrate having a metal coating to immobilize said DNA strands on said metal surface.
A method of manufacturing the substrate of claim 3 in which DNA strands having a double-strand portion and a single-strand portion, with a thiol group being present on the terminal of said double-strand portion, are contacted with the surface of a glass substrate or silicon substrate that has been surface treated with a hetero bifunctional crosslinking agent, to immobilize said DNA strands on said surface.
A method of manufacturing the substrate of claim 7 in which DNA strands having a double-strand portion and a single-strand portion, with a thiol group being present on the terminal of said double-strand portion, and DNA strands comprised of only a double-strand portion on the terminal of which a thiol group is present are contacted with the surface of a glass substrate or silicon substrate that has been surface treated with a hetero bifunctional crosslinking agent to immobilize said DNA strands on said surface.
The method of manufacturing according to claim 12 or claim 13 wherein said hetero bifunctional crosslinking agent is at least one member selected from the group consisting of: succinimidyl-4-[maleimidophenyl]butyrate (SMPB), m-maleimidobenzoyl-N-hydroxysuccinimidoester (MBS), succinimidyl-4-(maleimidomethyl)cyclohexane-1-carboxylate (SMCC), N-(gamma-maleimidobutyloxy)succinimidoester (GMBS), m-maleimidopropionic acid-N-hydroxysuccinimidoester (MPS), and N-succinimidyl(4-iodoacetyl)amino benzoate (SIAB).
The method of manufacturing according to any of claims 10 to 14 wherein the contact with the DNA strands is conducted in the presence of bivalent metal ions.
The method of manufacturing according to claim 15 wherein said bivalent metal ions are magnesium ions.
A method in which target DNA is contacted with the surface of any of the substrates of claims 1 to 9 on which DNA strands have been immobilized to test for complementarity between the target DNA and the single-strand portion of said DNA strands having a double-strand portion and a single-strand portion.
The method according to claim 17 wherein the contacting of target DNA with a surface on which DNA strands have been immobilized is conducted in the presence of bivalent metal ions.
The method according to claim 18 wherein said bivalent metal ions are magnesium ions.
The method according to any of claims 17 to 19 wherein the presence or absence of hybridization between the target DNA and the single-strand portion of said DNA strands is detected by the surface plasmon resonance method or quartz crystal microbarance (QCM) method.
The method according to any of claims 17 to 19 wherein said target DNA is DNA having a fluorescent label and hybridization with said the single-strand portion of said DNA strand is detected by means of fluorescence or Radio Isotope (RI).
The method according to any of claims 17 to 21 wherein target DNA comprising a mismatched nucleic acid base is detected.

### Brief Description of the Drawings

Fig. 1 shows the scheme of preparing thiolated DNA oligomer (DNA strand comprising a double-strand portion and a single-strand portion (50mer/20mer SH complex (1)) and DNA strand comprising only a double-strand portion (20mer C/ 20mer SH complex (2)), immobilization thereof on a substrate, and hybridization conducted in the Example.
Fig. 2 shows the results of *in site* observation by surface plasmon resonance of immobilization on a metal substrate surface when the ratio of 50mer/20mer SH complex (1) to 20mer C/20mer SH complex (2) was varied from 0:100, 50:50, to 100:0.
Fig. 3 shows the results of *in site* observation by surface plasmon resonance of the hybridization of target DNA 5'-CTGTGTCGATCAGTTCTCCA-3' (20mer M) to the substrate.
Fig. 4 shows the results of *in site* observation by surface plasmon resonance of the hybridization of target DNA 5'-CTGTGTCGATCAGTTCTCCA-3' (20mer M) and control DNA 5'-CTGTGTCAATCAGTTCTCCA-3' (20mer S) having an only one nucleic acid difference in a nucleotide sequence.

### Best Modes of Implementing the Invention

### Substrate

The hybridization substrate of the present invention is characterized in that DNA strands having a double-strand portion and a single-strand portion are immobilized on a substrate surface, with the double-strand portion side of said DNA strand being immobilized to said substrate surface.

In conventional substrates on which DNAs are immobilized, the DNAs are single-stranded. By contrast, DNA strands comprising a double-strand portion and a single-strand portion are employed in the present invention. That is, the DNA strands employed in the present invention respectively comprises one strand that is longer than the other, with one portion being double-strand and the other portion being single-strand. The DNA strand employed in the present invention starts out as a double-strand portion, becoming a single-strand portion along the way and remaining so to the end. The number of nucleotides of the double-strand portion and the number of nucleotides of the single-strand portion are not specifically limited. However, the number of nucleotides of the double-strand portion can range from 10 to 80 to achieve stability of the double-strand portion, and the number of nucleotides of the single-strand portion can range from 20-90 to facilitate movement of the single-strand portion.

This DNA strand is comprised of two single strands of differing length, where the nucleotide sequence of the shorter single-strand DNA is complementary to the nucleotide sequence from either end of the longer single-strand DNA, and is manufactured by hybridizing the two single strands.

In the substrate of the present invention, the DNA strands having a double-strand portion and single-strand portion are immobilized on the substrate surface on the double-strand portion side. Such a configuration affords the advantages of having the double-strand portion near the substrate surface with the single-strand portion of the DNA strands being removed from the substrate surface, resulting in a certain space around the single-strand portion, facilitating use of the single-strand portion as a hybridization sequence, and placing the double-strand portion in close proximity to the substrate surface to prevent horizontal collapsing of the DNA strands.

When the substrate is a metal substrate or a substrate having a metal coating, the DNA strands can be immobilized on the substrate surface, for example, by immobilization with a sulfur atom on the metal surface of the substrate. Examples of metal substrates are gold, silver, chromium, gallium, nickel, and neodymium. Examples of substrates having metal coatings are glass, mica, and similar substrates with surface coatings of gold, silver, chromium, gallium, nickel, neodymium, and the like.

The immobilization of the DNA strands to the metal surface of the substrate by means of a sulfur atom will be described in detail in the method of manufacturing substrates.

When the substrate is a glass or silicon substrate, the DNA strands can be immobilized by means of sulfur atoms to the substrate surface. An example of a glass substrate is a substrate of common slide glass. An example of a silicon substrate is a silicon wafer.

The immobilization of the DNA strands to the surface of the substrate by means of a sulfur atom will be described in detail in the method of manufacturing substrates.

In the substrate of the present invention, in addition to the above-described DNA strands having a double-strand portion and a single-strand portion, DNA strands having only a double-strand portion may also be immobilized on the surface of the substrate. Immobilization of DNA strands having only a double-strand portion on the surface of the substrate affords the advantages of increasing the space around the single-strand portion at a spot removed from the substrate surface, facilitating use of the single-strand portion as a hybridization sequence, and causing the double-strand portion to be densely present near the substrate surface, preventing horizontal collapsing of the DNA strand.

The substrate may be a metal substrate or a substrate having a metal coating. In that case, in addition to DNA strands having a double-strand portion and a single-strand portion, DNA strands having only a double-strand portion can be immobilized on the metal surface of the substrate by means of a sulfur atom. The substrate may also be a glass or silicon substrate. In that case, in addition to DNA strands having a double-strand portion and a single-strand portion, DNA strands having only a double-strand portion may be immobilized on the surface of the substrate by means of a sulfur atom.

When DNA strands having only a double-strand portion are immobilized on the substrate surface in addition to DNA strands having a double-strand portion and a single-strand portion as set forth above, the ratio of the number of immobilized DNA strands having a double-strand portion and a single-strand portion to the number of immobilized DNA strands having only a double-strand portion can be suitably determined based on the density (compactness) of the single strand portion, which is the sequence employed in hybridization. Examples of suitable ratios are from 99:1 to 1:99, preferably from 99:1 to 25:75.

### Method of Manufacturing the Substrate

The substrate of the present invention can be manufactured, for example, in the case of a metal substrate or a substrate having a metal coating, by contacting DNA strands having a double-strand portion and a single-strand portion with a thiol group present on the terminal of the double-strand portion, with the metal surface of the substrate to immobilize the DNA strands on the metal surface. As stated above, double-strand DNA having a double-strand portion and a single-strand portion consists of two single strands of different length, where the shorter strand of single-strand DNA has a nucleotide sequence complementing the nucleotide sequence of the longer strand of single-strand DNA from one of the ends thereof, and is manufactured by hybridizing the two single strands. For example, a thiol group is connected at the 5' end of a short single strand of DNA and this sequence of short single-strand DNA is hybridized with a long single strand of DNA having a complementary sequence on its 3' end to obtain a DNA strand having a double-strand portion and a single-strand portion with the double-strand portion having a terminal thiol group. A thiol group may be incorporated at the 5' terminal of the single-strand DNA by a known C6 synthesis method (for example, see Chemistry and Biology Experiments line 22, Tamba, Mineo, "DNA Chemical Synthesis", pp. 38-43, Hirokawa Shoten).

Hybridization of the short single-strand DNA sequence and the long single-strand DNA having a complementary sequence from the 3' end may also be suitably conducted by the usual methods under the usual conditions.

The DNA strand may be immobilized on the metal surface of the substrate by contacting the DNA strand having a double-strand portion, a single-strand portion, and a thiol group on the terminal of the double-strand portion with the metal surface. Known methods of immobilizing a DNA strand having a thiol group are described, for example, in J. Am. Chem. Soc. 1998, 120, 9787-9792.

A mixture of a prescribed ratio of DNA strands having a double-strand portion, a single-strand portion, and a thiol group on the terminal of the double-strand portion to DNA strands having only a double-strand portion comprising a terminal thiol group can be contacted with the metal surface of a metal substrate or substrate having a metal coating in the same manner as set forth above to immobilize DNA strands having a double-strand portion and a single-strand portion and DNA strands having only a double-strand portion on the metal surface in a prescribed ratio.

When the substrate of the present invention is glass or silicon, for example, the substrate surface can be treated with a hetero bifunctional crosslinking agent, and the treated surface can be contacted with DNA strands having a double-strand portion, a single-strand portion, and a terminal thiol group on the double-strand portion to immobilize the DNA strands on the surface. DNA strands having a double-strand portion, single-strand portion, and terminal thiol group on the double-strand portion can be manufactured by the above-described method.

When incorporating a thiol group onto the terminal of the double-strand portion of the DNA strand having a double-strand portion and a single-strand portion described above, the thiol group may be incorporated onto either the short strand or the long strand of the DNA strand having a double-strand portion and a single-strand portion. This is because it is advantageous that the long strand having a single-strand portion contributing to hybridization can be used without any treatments other than hybridization with the short strand.

This method of immobilization is described in, for example, Nucleic Acids Research, 1996, Vol. 24, No. 15, 3031-3039.

The hetero bifunctional crosslinking agent may be at least one member selected from the group consisting of: succinimidyl-4-[maleimidophenyl]butyrate (SMPB), m-maleimidobenzoyl-N-hydroxysuccinimidoester (MBS), succinimidyl-4-(maleimidomethyl)cyclohexane-1-carboxylate (SMCC), N-(gammamaleimidobutyloxy)succinimidoester (GMBS), m-maleimidopropionic acid-N-hydroxysuccinimidoester (MPS), and N-succinimidyl(4-iodoacetyl)amino benzoate (SIAB).

The mixture comprising a prescribed ratio of DNA strands having a double-strand portion, a single-strand portion, and a thiol group on the terminal of the double-strand portion to DNA strands comprised of only a double-strand portion having a terminal thiol group can be contacted with the surface of a glass substrate or silicon substrate that has been treated with the hetero bifunctional crosslinking agent to immobilize the two types of DNA strands on the surface in a prescribed ratio.

The DNA strands having a double-strand portion, single-strand portion, and terminal thiol group on the double-strand portion, or the mixture comprising a prescribed ratio of DNA strands having a double-strand portion, a single-strand portion, and a thiol group on the terminal of the double-strand portion and DNA strands having only a double-strand portion having a terminal thiol group, are desirably contacted with the metal surface of the substrate in the presence of a bivalent metal ion in the method of immobilizing the DNA strand on the metal surface. Immobilization in the presence of bivalent metal ions affords the advantages of increasing the stability of the double-strand portion, aggregating the double-strand portion, and rendering it stable on the substrate.

Examples of bivalent metal ions are magnesium ions, calcium ions, cobalt ions, barium ions, strontium ions, cadmium ions, zinc ions, and iron ions. Suitable concentrations of these bivalent ions range from 1 to 1,000 mM.

Similarly, for the above-stated reasons, the method of contacting the DNA strands having a double-strand portion, single-strand portion, and terminal thiol group on the double-strand portion, or the mixture comprising a prescribed ratio of DNA strands having a double-strand portion, a single-strand portion, and a thiol group on the terminal of the double-strand portion and DNA strands having only a double-strand portion having a terminal thiol group with the surface of a substrate that has been treated with a hetero bifunctional crosslinking agent to immobilize the DNA strands on the surface, is also desirably conducted in the presence of bivalent metal ions.

### Method of Testing Complementarity

The complementarity test method of the present invention comprises the contacting of target DNA with the surface of the substrate of the present invention on which DNA strands have been immobilized, and testing the complementarity of the single-strand portion of the DNA strand having a double-strand portion and a single-strand portion with the target DNA. More specifically, a suitably means is employed to detect the presence of target DNA that has hybridized with the single-strand portion of the DNA strand having a double-strand portion and a single-strand portion to test for complementarity.

Known methods may be suitably employed to detect the presence or absence of hybridization between the target DNA and the single-strand portion of the DNA strand. Examples of detection methods are the surface plasmon resonance method and the quartz crystal microbarance (QCM) method.

In the surface plasmon resonance method, the substrate surface is exposed to a laser beam and the resonance of plasmon generated on the substrate surface is detected to measure the thickness of the film or the like present on the substrate surface. The presence or absence of DNA strand that has hybridized with the target DNA is identified by the difference in film thickness to determine the presence or absence of hybridization.

In the surface plasmon resonance method, there is no need to label the DNA strand immobilized on the substrate or the target DNA, permitting convenient detection of the presence or absence of hybridization.

The quartz resonator method is a method in which the reduction in frequency due to adhesion of compounds to a quartz resonator electrode is used to determine the mass of the adhering product (for example, see Chem. Rev., 1992, 92, 1355-1379).

The presence or absence of hybridization between the target DNA and the single-strand portion of DNA strand can be detected by, for example, employing DNA having a fluorescent label as target DNA and detecting the presence or absence of hybridization with the single-strand portion of the DNA strand based on fluorescence. Methods of detecting the presence or absence of fluorescent-labeled DNA and hybridization based on fluorescence are known. In the present invention, these known techniques may be employed unaltered.

Target DNA comprising a mismatched nucleic acid base may also be detected by the complementarity test method of the present invention. That is, in the complementarity test method of the present invention, completely complementary target DNA hybridizes, while target DNA having a single mismatched nucleic acid base does not hybridize, permitting the detection of target DNA comprising a single mismatched nucleic acid base.

In the complementarity test method of the present invention, the target DNA is desirably contacted with the surface on which the DNA strand has been immobilized in the presence of bivalent metal ions in order to stabilize the double-strand portion following hybridization. Examples of bivalent metal ions are: magnesium ions, calcium ions, cobalt ions, barium ions, strontium ions, cadmium ions, zinc ions, and iron ions. Suitable concentrations of these bivalent metal ions range from 1 to 1,000 mM, for example.

Modes of immobilizing DNA strand on the substrate have been described in the present invention. However, either the double-strand portion or the single strand portion may be RNA or PNA.

Further, either of the single strands of the strand comprising only a double-strand portion may be RNA or PNA.

### Examples

The present invention is further described below in view of the examples.

### Example 1

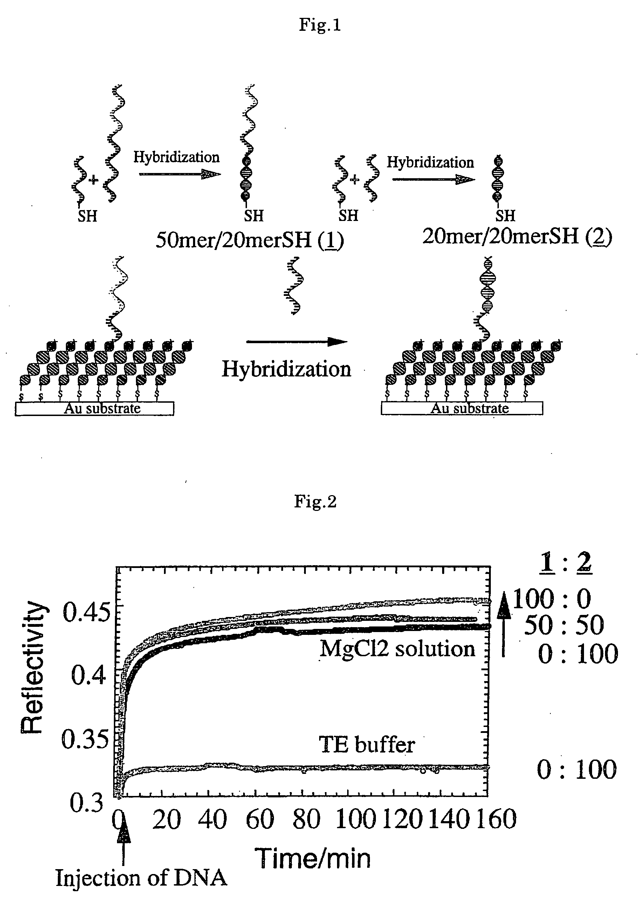

Based on the scheme shown in Fig. 1, thiolated DNA oligomer (DNA strand comprising a double-strand portion and a single-strand portion (50mer and 20mer double-strand DNA (1)) and DNA strand comprising only a double-strand portion (20mer and 20mer double-strand complex (2)) were prepared, immobilized on a substrate, and employed in hybridization.

### Synthesis of Thiolated DNA Oligomer

HS-5'-ATGCATGCATTAGCATGCTA-3' (20mer SH) thiolated at the 5' terminal was synthesized by the known C6 synthesis method (for example, see Chemistry and Biology Experiments line 22, Tamba, Mineo, "DNA Chemical Synthesis", pp. 38-43, Hirokawa Shoten).

### Immobilization on the Metal Surface of Thiolated Double-Strand DNA Oligomer

The HS-5'-ATGCATGCATTAGCATGCTA-3' (20mer SH) synthesized above and
5'-TAGCATGCTAATGCATGCATTTTTTTTTTTTGGAGAACTGATCGACACAG-3'
(50mer) were superheated to 95°C in a buffer solution and slowly cooled to form probe DNA in the form of partial double-helix 50mer/20mer SH complex (1).

20mer C/20mer SH complex (2) of 5'-TAGCATGCTAATGCATGCAT-3' (20mer) and 20mer SH was formed by the same procedure and employed as a mixture diluting compound.

50mer/20mer SH complex (1) and 20mer C/20mer SH complex (2) were employed to immobilize on a gold substrate surface double-strand DNA oligomer that had been thiolated under the conditions given below:
Buffer: MgCl₂(H₂O)₆ was adjusted to a concentration of 20 mM and the solution was sterilized for 20 h at 120°C and employed as solvent.
Concentration of thiolated double-strand DNA oligomer: about 3.0 micromoles/liter.
Temperature: 20°C
After-treatment: Rinsing with solvent and washing away of excess DNA.

### Hybridization Test

Target DNA was hybridized with the above substrate on which the double-strand DNA oligomer had been immobilized.

5'-CTGTGTCGATCAGTTCTCCA-3' (20mer M) expected to hybridize complementarily with the probe site of the probe DNA was employed as target DNA. Further, to test detection of the tautomeric structure (nucleic acid mismatching) of these nucleotides, 5'-CTGTGTCAATCAGTTCTCCA-3' (20mer S) with a sequence differing by only one nucleic acid from 20mer M was employed as control DNA.

A 20 mM MgCl₂ aqueous solution was employed as solvent to dissolve the DNA for hybridization. Hybridization was conducted for 2 h at a temperature of 20°C.

### Specific Methods of Evaluating Monomolecular DNA Films

Hybridization of target DNA adsorbed onto the solid metal substrate and probe DNA immobilized on the substrate was observed by surface plasmon resonance.

Further, the DNA monolayer film was evaluated by atomic force microscopy (AFM), photoelectric spectroscopy (XPS), and infrared reflection absorption spectroscopy (IR-RAS).

### Confirmation of DNA Immobilization

The state of immobilization on the gold substrate surface when the ratio of 50mer/20mer SH complex (1) to 20mer C/20mer SH complex (2) was varied from 0:100, 50:50, to 100:0 was observed by surface plasmon resonance; the results are given in Fig. 2. When MgCl₂-comprising buffer was employed, immobilization of the complex on the gold substrate surface was confirmed at all ratios.

### Confirmation of Hybridization

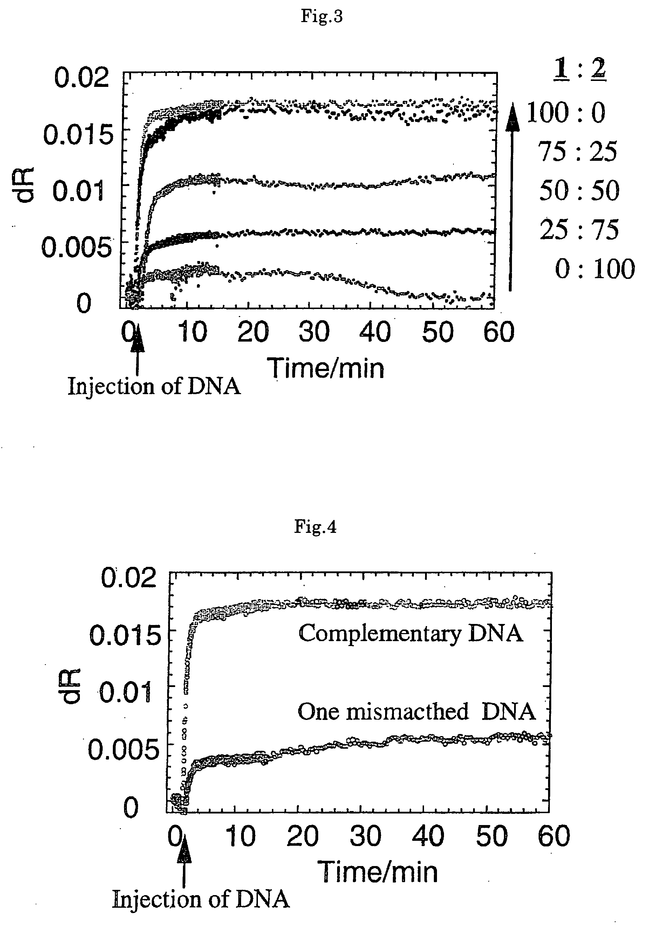

The ratio of above-described 50mer/20mer SH complex (1) to 20mer C/20mer SH complex (2) was varied from 0:100, 25:75, 50:50, 75:25, to 100:0 and hybridization of target DNA in the form of 5'-CTGTGTCGATCAGTTCTCCA-3' (20mer M) on a substrate obtained by immobilizing these complexes on a gold substrate surface was observed by surface plasmon resonance. The results are given in Fig. 3. The higher the ratio of 50mer/20mer SH complex (1), the greater the hybridization rate achieved. Nearly identical results were obtained for ratios of 75:25 and 100:0.

### Confirmation of Single-Nucleic Acid Mismatching

Above-described 50mer/20mer SH complex (1) and 20mer C/20mer SH complex (2) were immobilized on the surface of a gold substrate in a ratio of 100:0 and the state of hybridization of target DNA 5'-CTGTGTCGATCAGTTCTCCA-3' (20mer M) and a control DNA 5'-CTGTGTCAATCAGTTCTCCA-3' (20mer S) sequence differing by one nucleic acid on this substrate was observed by surface plasmon resonance. The results are given in Fig. 4. It was found that a mismatch of one nucleic acid could be identified by the method of the present invention.

The present invention provides a hybridization substrate on the surface of which DNA strands have been immobilized and that exhibits increased surface coverage and activity as well as a manufacturing method for the same. The present invention further provides a complementarity test method employing this substrate.

## Claims

1. A hybridization substrate in which DNA strands having a double-strand portion and a single-strand portion are immobilized on a substrate surface, wherein the double-strand portion side of said DNA strands is immobilized on said substrate surface.

2. The substrate according to claim 1 wherein said substrate is a metal substrate or a substrate having a metal coating, and said DNA strands have been immobilized on the metal surface of said substrate by means of a sulfur atom.

3. The substrate according to claim 1 wherein said substrate is a glass or silicon substrate and said DNA strands have been immobilized on the surface of said substrate by means of a sulfur atom.

4. The substrate according to any of claims 1 to 3 wherein the number of nucleic acids of said double-strand portion ranges from 10 to 80, and the number of nucleic acids of said single-strand portion ranges from 20 to 90.

5. The substrate according to any of claims 1 to 4 wherein in addition to said DNA strands having a double-strand portion and a single-strand portion, DNA strands having only a double-strand portion have been further immobilized on the surface of said substrate.

6. The substrate according to claim 5 wherein said substrate is a metal substrate or a substrate having a metal coating and said DNA strands having only a double-strand have been immobilized on said metal surface of said substrate by means of a sulfur atom.

7. The substrate according to claim 5 wherein said substrate is a glass substrate or silicon substrate and said DNA strands having only a double-strand portion have been immobilized on the surface of said substrate by a sulfur atom.

8. The substrate according to any of claims 5 to 7 wherein the immobilization ratio of said DNA strands having a double-strand portion and a single-strand portion to DNA strands having only a double-strand portion ranges from 99:1 to 1:99.

9. The substrate according to claim 2 or claim 6 wherein said metal substrate or metal coating is made of gold.

10. A method of manufacturing the substrate of claim 2 in which DNA strands having a double-strand portion and a single-strand portion, with a thiol group being present on the terminal of said double-strand portion, are contacted with the metal surface of a metal substrate or substrate having a metal coating to immobilize said DNA strands on said metal surface.

11. A method of manufacturing the substrate of claim 6 in which DNA strands having a double-strand portion and a single-strand portion, with a thiol group being present on the terminal of said double-strand portion, and DNA strands comprised of only a double-strand portion on the terminal of which a thiol group is present are contacted with the metal surface of a metal substrate or substrate having a metal coating to immobilize said DNA strands on said metal surface.

12. A method of manufacturing the substrate of claim 3 in which DNA strands having a double-strand portion and a single-strand portion, with a thiol group being present on the terminal of said double-strand portion, are contacted with the surface of a glass substrate or silicon substrate that has been surface treated with a hetero bifunctional crosslinking agent, to immobilize said DNA strands on said surface.

13. A method of manufacturing the substrate of claim 7 in which DNA strands having a double-strand portion and a single-strand portion, with a thiol group being present on the terminal of said double-strand portion, and DNA strands comprised of only a double-strand portion on the terminal of which a thiol group is present are contacted with the surface of a glass substrate or silicon substrate that has been surface treated with a hetero bifunctional crosslinking agent to immobilize said DNA strands on said surface.

14. The method of manufacturing according to claim 12 or claim 13 wherein said hetero bifunctional crosslinking agent is at least one member selected from the group consisting of: succinimidyl-4-[maleimidophenyl]butyrate (SMPB), m-maleimidobenzoyl-N-hydroxysuccinimidoester (MBS), succinimidyl-4-(maleimidomethyl)cyclohexane-1-carboxylate (SMCC), N-(gamma-maleimidobutyloxy)succinimidoester (GMBS), m-maleimidopropionic acid-N-hydroxysuccinimidoester (MPS), and N-succinimidyl(4-iodoacetyl)amino benzoate (SIAB).

15. The method of manufacturing according to any of claims 10 to 14 wherein the contact with the DNA strands is conducted in the presence of bivalent metal ions.

16. The method of manufacturing according to claim 15 wherein said bivalent metal ions are magnesium ions.

17. A method in which target DNA is contacted with the surface of any of the substrates of claims 1 to 9 on which DNA strands have been immobilized to test for complementarity between the target DNA and the single-strand portion of said DNA strands having a double-strand portion and a single-strand portion.

18. The method according to claim 17 wherein the contacting of target DNA with a surface on which DNA strands have been immobilized is conducted in the presence of bivalent metal ions.

19. The method according to claim 18 wherein said bivalent metal ions are magnesium ions.

20. The method according to any of claims 17 to 19 wherein the presence or absence of hybridization between the target DNA and the single-strand portion of said DNA strands is detected by the surface plasmon resonance method or quartz crystal microbarance (QCM) method.

21. The method according to any of claims 17 to 19 wherein said target DNA is DNA having a fluorescent label and hybridization with said the single-strand portion of said DNA strand is detected by means of fluorescence or Radio Isotope (RI).

22. The method according to any of claims 17 to 21 wherein target DNA comprising a mismatched nucleic acid base is detected.
